# EUROPEAN PATENT APPLICATION

(11) **EP 0 747 022 A2**
(43) Date of publication of application: **11.12.1996**
(21) Application number: 96304230.4
(22) Date of filing: 06.06.1996
(51) Int. Cl.: A61F 2/06

(54) **Coil-reinforced retractable sleeve for stent delivery catheter**

(30) Priority: 07.06.1995 US 480578
(71) Applicant: ADVANCED CARDIOVASCULAR SYSTEMS, INC., Santa Clara California 95052 (US)
(72) Inventor: Klemm, Kurt, Santa Clara, California 95051 (US)
(74) Representative: Mayes, Stuart David

(57) **Abstract**

A coil-reinforced retractable sleeve that provides a stent delivery system with improved longitudinal stiffness, lateral flexibility and overall pushability while maintaining dimensional stability. The coil-reinforced retractable sleeve comprising a spiral-wound ribbon encapsulated between an inner and an outer laminate which form an elongated tubular body adapted to slidably receive an intravascular catheter and having a first end adapted to connect with a manipulating device for effecting relative longitudinal movement of the intravascular catheter and sleeve and having a second end adapted for egress and ingress of the intravascular catheter.

## Description

### BACKGROUND OF THE INVENTION

This invention relates to devices for treatment of heart diseases and, more particularly, to a retractable sleeve for use in a stent delivery system.

Several interventional treatment modalities presently are used for heart disease including balloon and laser angioplasty, atherectomy and by-pass surgery. In typical balloon angioplasty procedures, a guiding catheter having a preformed distal tip is percu-taneously introduced through the femoral artery into the cardiovascular system of a patient in a conventional Seldinger technique and advanced within the cardiovascular system until the distal tip of the guiding catheter is seated in the ostium of a desired coronary artery. A guidewire is positioned within an inner lumen of a dilatation catheter and then both are advanced through the guiding catheter to the distal end thereof. The guidewire first is advanced out the distal end of the guiding catheter into the coronary vasculature until the distal end of the guidewire crosses a lesion to be dilated, then the dilatation catheter having an inflatable balloon on the distal portion thereof is advanced into the coronary anatomy of the patient over the previously introduced guidewire until the balloon and the dilatation catheter properly are positioned across the lesion.

Once in position across the lesion, the balloon, which is made of relatively inelastic materials, is inflated to predetermined size with radiopaque liquid at a relatively high pressure (e.g., greater than 0.41 bars (4 atmospheres) to compress the arteriosclerotic plaque of the lesion against the inside of the arterial wall and to otherwise expand the inner lumen of the artery. The balloon then is deflated so that the blood flow can be resumed through the dilatated artery and the dilatation catheter can be removed therefrom. Further details of dilatation catheters, guidewires, and devices associated therewith for angioplasty procedures can be found in U.S. Patent 4,323,071 (Simpson, et al.); U.S. Patent 4,554,929 (Sampson, et al.); U.S. Patent 4,616,652 (Simpson); U.S. Patent 4,638,805 (Powell); and U.S. Patent 4,748,982 (Horzewski, et al.).

A major focus of recent development work in the treatment of heart disease has been directed to endoprosthetic devices called stents. Stents are generally cylindrically-shaped intravascular devices which are placed within a damaged artery to hold it open. Stents can be used to prevent restenosis and to maintain the patency of a blood vessel immediately after intravascular treatments. In some circumstances, stents also can be used as the primary treatment device when they are expanded to dilate a stenosis and then left in place.

The rapid and effective delivery of a stent to a designated location within the vasculature of the patient is desirable, particularly when the stent is to be delivered within a coronary artery. That is, quickly placing a stent within the vasculature is desirable because the intrusive nature of the angioplasty procedure and the associated danger to the patient then may be minimized. It has been found to be difficult to quickly deliver a stent, however, particularly in those situations in which an intimal flap has occluded an artery.

Attempts to advance and place a stent in regions of coronary arteries occluded by dissected arterial linings have had varying degrees of success. A successful method for rapidly and effectively delivering a stent involves placing a compressed or otherwise small diameter stent about an expandable member, such as a balloon, on the distal end of an intravascular catheter and slidably disposing the intravascular catheter within an elongated sheath or sleeve having a distal port through which the catheter may egress. Thereafter, the sleeve and catheter may be advanced through the vascular system until the combination is in the desired location within a blood vessel and the relative axial positions of the sleeve and catheter may be manipulated so that the entire length of the stent mounted on the distal extremity of the catheter is emitted from the sleeve. Next, the balloon catheter may be expanded so as to seat the stent within the blood vessel. Finally, the balloon catheter is deflated and the sleeve and catheter are withdrawn, leaving the expanded stent within the blood vessel holding open the passageway thereof.

However, during advancement through the arduous turns of the vascular anatomy, the sheath or sleeve and catheter of conventional stent delivery systems have a tendency to kink or buckle. Kinking or buckling not only impedes the ability to move the system forward to the treatment site, but also can cause damage to the system or to the vessels of the patient. If a stent delivery system cannot be further advanced, it must be removed from the patient and replaced with a new system which hopefully will not exhibit the same problem.

Such kinking or buckling generally is attributed to the stiffness of the delivery systems. Although some degree of longitudinal stiffness certainly is desirable to enhance the ease with which the system can be pushed through the vasculature, a lack of flexibility in the lateral direction impedes the capacity of the system to accommodate the often sharp twists and turns of the vessels as the distal end of the system is advanced further and further towards the heart or other targeted treatment site.

In addition to stiffness, it also is important that the material used in the portion of a stent delivery system that extends out of the guiding catheter and into the coronary anatomy in which treatment is to be rendered does not have the effect of increasing the cross-sectional profile of that portion. It is critical, of course, to provide this section with a low enough profile to allow the stent to be delivered into the very small diameter vessels of the heart.

In order to optimize the requirements of stiffness and low profile, a homogeneous material typically is selected for the distal portion of conventional stent delivery systems. Such a material, however, often lacks sufficient lateral flexibility, and thereby the risk of kinking and buckling is not avoided. In fact, the magnitude of this risk increases as the system is advanced, in correspondence with the increase in tortuosity of the anatomy as the stent approaches the delivery site.

Due to the nature of the problems that stents are designed to treat, it obviously is of paramount importance to have the capability of rapidly and effectively deploying the intravascular devices. Therefore, a stent delivery system having improved stiffness, pushability, and flexibility is most desirable.

Accordingly, what is needed and what heretofore has been unavailable is a stent delivery system comprising a structure that resists kinking and buckling while being advanced through the vasculature of a patient, without compromising the desired characteristics of the pushability and low-profile of the system.

### SUMMARY OF THE INVENTION

Embodiments of the present invention provide a coil-reinforced retractable sleeve for use in a stent delivery system, which contributes to the ability of the system to resist kinking or buckling while it is being advanced, and which provides the stent delivery system with improved longitudinal stiffness, improved lateral flexibility, and greatly enhanced pushability and retraction response, while maintaining dimensional stability and the requisite low profile. By comprising greatly enhanced pushability and retraction response, a one-to-one ratio is approached between the degree to which the delivery system is advanced into a patient's vasculature of a stent delivery system at the proximal end and the degree to which the distal end of the system bearing the stent is advanced to the site at which treatment is to be rendered.

In a preferred embodiment, a coil-reinforced retractable sleeve may embody an elongated tubular body comprising a spiral-wound ribbon encapsulated between two laminates, and may be produced using a manufacturing process that assures dimensional stability. It is contemplated that the laminated coil-reinforced structure comprises a substantial portion of the retractable sleeve, and that this portion extend distally from a manipulating device (for causing axial motion of the retractable sleeve) to a point proximal a distal end of the sleeve. The remaining or distal portion of the coil-reinforced retractable sleeve is contemplated to comprise another material.

It also is contemplated that the spiral-wound ribbon comprise relatively inelastic material, so that each adjacent turn of the spiral translates an applied longitudinal force into advancing progression of the system into the patient, without causing an elastic deformation of the ribbon material, thereby resulting in structure having improved stiffness. Further, the ribbon preferably is formed to have a thin cross-section in a generally rectangular shape, in order to minimize the effect the multi-layer structure has on the overall profile of the stent delivery system.

The spiral-wound ribbon may comprise a relatively large number of turns, so that kinking and buckling can be resisted without substantially impeding lateral flexibility, thereby resulting in a structure having improved flexibility. Moreover, by selecting a laminate, such as polyimide, a substance that inherently resists buckling and kinking, the sleeve consequently exhibits superior flexibility. By comprising improved longitudinal stiffness and lateral flexibility, the sleeve necessarily possesses improved pushability and retraction response.

In addition, in a preferred embodiment the tubular, coil-reinforced retractable sleeve may be adapted to slidably receive an intravascular catheter, such as a balloon catheter, for delivering a stent to a designated area within a blood vessel and may further comprise a distal port through which the intravascular catheter may egress. Moreover, the sleeve may be adapted to connect to a manipulating device, for retracting the sleeve or for effecting relative longitudinal movement of the sleeve and intravascular catheter.

In another embodiment, the laminated coil-reinforced structure may comprise the entire length of the retractable sleeve and may further comprise a distal end that tapers down in a substantially sphere-like manner so that the cross-sectional area is somewhat less in the distal region than the cross-sectional area of the rest of the sleeve. A coil- reinforced retractable sleeve having a tapered distal end comprises a profile well suited for traveling through the vasculature of a patient. The sleeve also may incorporate a notch at its distal end for providing a softer and more flexible structure where desired. Further, the sleeve may embody a proximal port that is disposed in a wall of the retractable sleeve proximate to the distal port and that is adapted to receive a guidewire. It also is contemplated that the sleeve comprise a slot extending from the proximal port to a location just proximate to the distal port which functions to facilitate relative movement of a guidewire through the retractable sleeve. Finally, the retractable sleeve may embody a plurality of slits formed in the wall of the sleeve which slits extend proximally from the distal port and which operate to enable the distal end to be compressed to a smaller cross-sectional profile.

In yet another embodiment, it is contemplated that the coil-reinforced retractable sleeve further comprise a thin-walled material that encapsulates the inner and outer laminates. The thin-walled material functions to add strength to the sleeve as well as to resist kinking and its effects.

Other aspects and advantages of the invention will become apparent from the following detailed description, taken in conjunction with the accompanying drawings, which illustrate, by way of example the principles of the invention.

### BRIEF DESCRIPTION OF THE DRAWINGS

FIGURE 1 is a partial longitudinal cross-sectional view of a stent delivery system which embodies the invention, illustrating a preferred embodiment of a coil-reinforced retractable sleeve.

FIG. 1a is a partial longitudinal cross-sectional view of a stent delivery system which embodies the invention, illustrating one embodiment of a coil-reinforced retractable sleeve.

FIG. 2 is a partial segmented view of a preferred embodiment of the invention, illustrating a portion of a coil-reinforced retractable sleeve having a wound ribbon interior encapsulated between an inner and an outer laminate.

FIG. 2a is a cross-sectional view taken along the lines 1-1 of FIG. 2, illustrating the flat cross-section of the wound ribbon.

FIG. 3 is a top view of another embodiment of the invention, illustrating a coil-reinforced sleeve comprising structure for expediting stent delivery.

FIG. 4 is a partial cross-sectional view of yet another embodiment of the invention, illustrating a coil-reinforced sleeve incorporating a notch for expediting stent delivery.

### DETAILED DESCRIPTION OF THE PREFERRED EMBODIMENTS

As is shown in the drawings, which are provided for purposes of illustration and not by way of limitation, the invention is embodied in a coil-reinforced retractable sleeve that resists kinking and buckling and provides improved stiffness, flexibility, retractability and pushability while maintaining overall dimensional stability. Stent delivery systems found in the art accomplish placement of stents within a blood vessel but typically comprise structure that buckles and kinks during advancement through the vasculature of a patient. Because the vasculature of a patient has many turns, a system that may advance through the vasculature while avoiding buckling and kinking is desirable. Further, because it is important to minimize the intrusive nature of delivering a stent within the vasculature, it is advantageous to have a stent delivery system comprising structure with improved stiffness, flexibility and pushability so that a stent may be quickly placed within a blood vessel. Therefore, the coil-reinforced retractable sleeve to be described is an improvement over conventional sleeves employed by stent delivery systems.

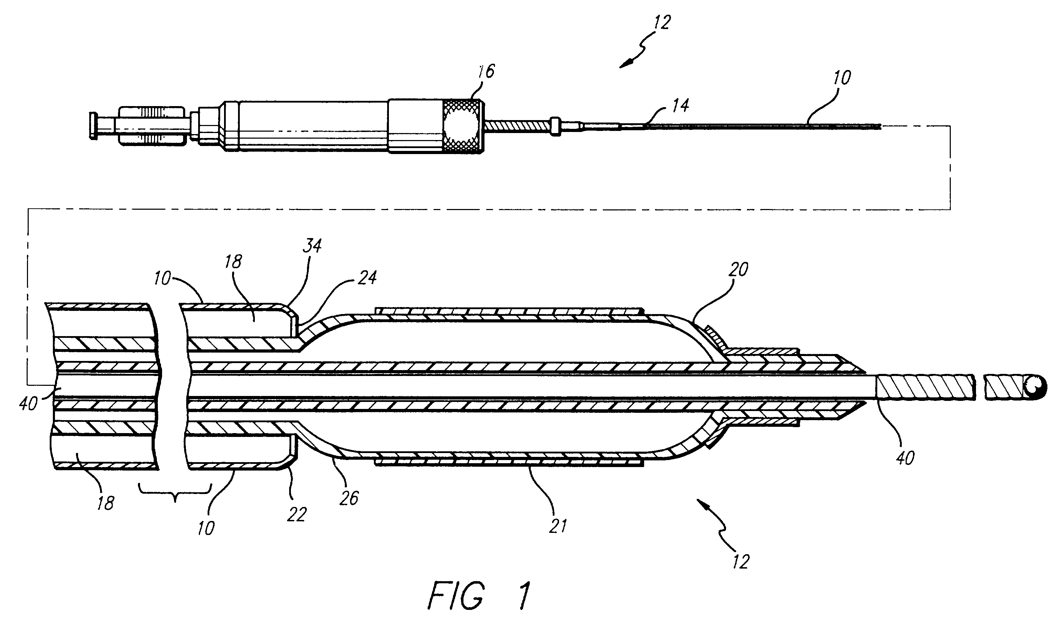

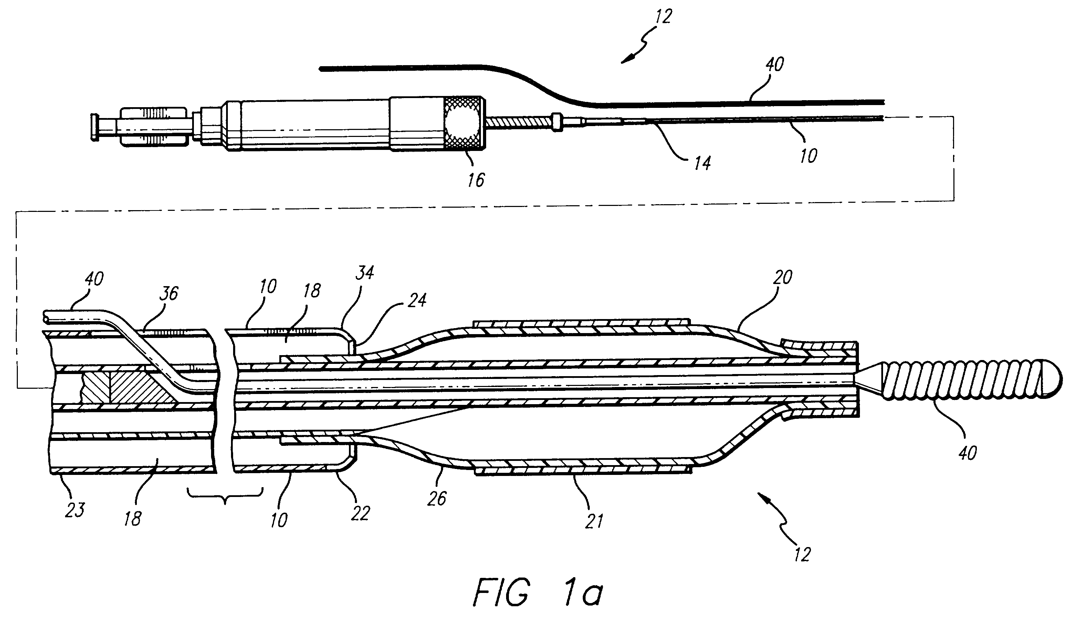

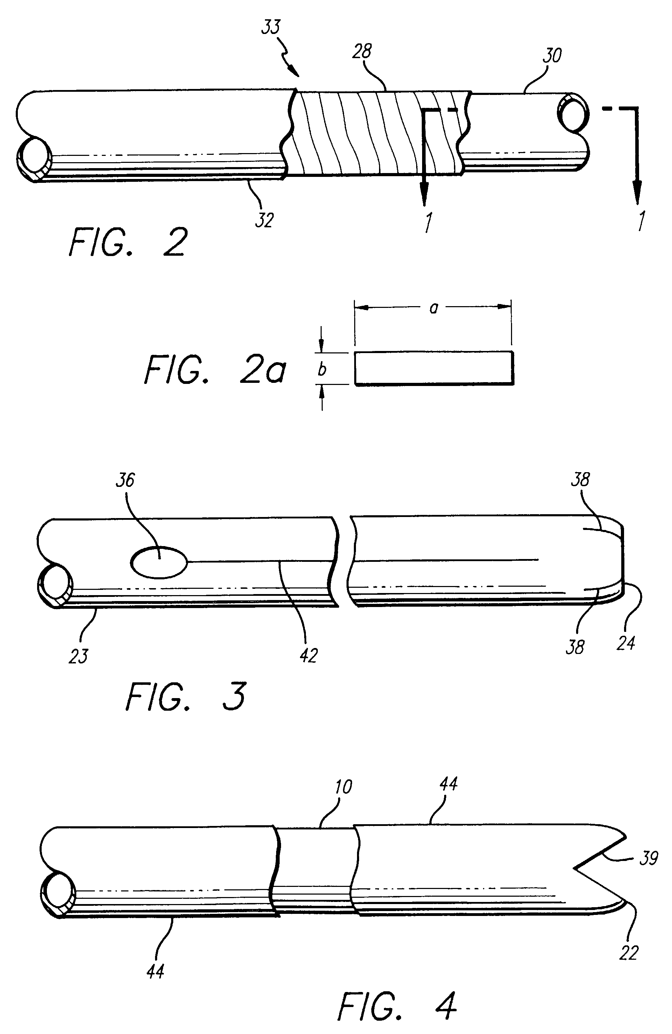

Referring to FIGS. 1 and 1a, the present invention is embodied in a coil-reinforced retractable sleeve 10 for use in a stent delivery system 12. In a preferred embodiment, and as can be seen in FIG. 2, it is contemplated that the sleeve 10 comprise a spiral-wound ribbon 28 encapsulated between an inner laminate 30 and an outer laminate 32, the ribbon and laminates together forming a coil-reinforced structure 33. It is also contemplated that the sleeve 10 comprise an elongated tubular body having a proximal end 14 adapted to connect to a manipulating device 16 for retracting the sleeve 10. Further, the sleeve may embody a hollow interior 18 adapted to slidably receive an intravascular catheter 20, such as a balloon catheter, for delivering a stent 21.

The laminated coil-reinforced structure 33 may or may not extend the length of sleeve 10. In the preferred embodiment, the laminated coil-reinforced structure extends to a point near a distal end 22 of the retractable sleeve 10. For example, the laminated coil-reinforced portion may extend to a point 23 just proximal to port 36 (See FIGS. 1a and 3). The remaining length of sleeve 10 may comprise polyethylene or an equivalent composition that may be attached to the laminated coil-reinforced portion 33 and may further comprise a distal end 22 having a distal port 24 formed therein. The distal port 24 may be of sufficient size to allow for, upon operation of the manipulating device 16, ingress and egress of an inflatable portion 26 of a balloon catheter 20 retaining a stent 21.

In the preferred embodiment, the spiral-wound ribbon 28 and inner laminate 30 and outer laminate 32 comprise a coil-reinforced struction 33 having a generally uniform cross-sectional area and constant diameter. As can be seen in FIG. 2a, the spiral-wound ribbon further is formed so as to have an almost flat cross-section, generally in the shape of a rectangle *a,b,* to prevent the presence of the ribbon from having any appreciable effect on the overall profile of the stent delivery system. The spiral-wound ribbon 28 may comprise any relatively inelastic material, for example any metal, that may be produced having the geometric configuration of a ribbon and that may be spiral-wrapped so as to take on a cylindrical form. It may be desirable to select a ribbon material with radiopaque characteristics.

Further, it is contemplated that the spiral-wound ribbon 28 comprise a relatively large number of turns per centimeter (per inch). For example, the spiral-wound ribbon 28 may have between 19.7 and 27.6 turns per centimeter (fifty and seventy windings per inch). It is to be understood that the more windings per centimeter or per inch of ribbon 28, the more flexible sleeve 10 becomes. By combining a relatively inelastic material and large number of turns per centimeter or per inch, ribbon 28 provides the sleeve 10 with improved longitudinal stiffness.

The laminates 30,32 also may comprise virtually any material that may be formed to encapsulate a spiral-wound ribbon. In a preferred embodiment, it is contemplated that the laminates 30,32 be made from polyimide. Polyimide is an appropriate material since it resists kinking and buckling as well as possesses processing and mechanical characteristics suited for forming and encapsulating a tubular structure.

In order to construct a sleeve 10 having dimensional stability, the sleeve 10 may be manufactured by first producing the ribbon 28 and then molding the laminates 30,32 about the ribbon 28. In the alternative, it may be desirable to incorporate the winding of a ribbon 28 into a process for extruding tubing. Thereafter, the sleeve 10 may be cut to a length useful for delivering a stent 21 within a desired location of a blood vessel.

In another embodiment, the laminated coil-reinforced portion extends the length of the sleeve 10 and further may comprise structure functioning to expedite the delivery of a stent within the vasculature of a patient. The sleeve 10 includes a tapered distal end 34 that narrows in a sphere-like manner and a proximal port 36 disposed in a wall proximate to the distal end 22 of the sleeve (See FIGS. la and 3). A plurality of slits 38 extending proximally from the distal end of the sleeve 22 and substantially parallel to a longitudinal axis of the sleeve 10. The slits 38 function to provide the stent delivery system 12 with a low profile well suited for traveling through a blood vessel. One or more notches 39 also may be incorporated into the distal end of the sleeve 22 in order to facilitate advancement of the stent delivery system 12 through a blood vessel (See FIG. 4). Slot 42 extends distally from the proximal port 36 and substantially parallel to the longitudinal axis of the sleeve 10. The proximal port 36 provides a through-hole for a guidewire 40 about which an intravascular catheter 20 and sleeve 10 travel and are guided to a desired location within the vasculature of a patient. Slot 42 extending from the proximal port 36 facilitates the relative motion of the sleeve 10 and the guidewire 40.

In yet another embodiment, the sleeve 10 further may be encapsulated with a thin-walled material (See for example, FIG. 4). By further encapsulating the sleeve 10 with a thin-walled material, for example shrink tubing, the sleeve 10 may possess additional material strength while substantially retaining its improved pushability and kink resistance capability.

It also is contemplated that the coil-reinforced structure of the sleeve 10 may be applied to all catheter shafts, inner members and outer members. In addition, it is contemplated that all catheter shafts, inner members and outer members as well as the sleeve solely comprise a polyimide material substantially having the pushability of a sleeve embodying a ribbon.

From the foregoing it will be appreciated that embodiments of the invention provide a coil-reinforced retractable sleeve 10 that resists kinking and buckling and that provides a stent delivery system with improved stiffness, flexibility, pushability and retraction response. As a stent delivery system incorporating the invention is advanced through the vasculature of a patient, the coil-reinforced retractable sleeve 10 resists kinking and buckling, thereby facilitating expeditious delivery of a stent within a blood vessel.

While several particular forms of the invention have been illustrated and described, it also will be apparent that various modifications can be made without departing from the scope of the invention. Thus, it should be understood that various changes in form, detail and application of the present invention may be made without departing from the spirit and scope of this invention.

## Claims

1. A stent delivery system, comprising:
an elongate tubular body, said elongate tubular body having a proximal portion and a distal portion;
said proximal portion defined by an inner laminate and an outer laminate;
a ribbon having a substantially flat cross-section, said ribbon being spirally wound about said inner laminate and encapsulated between said inner laminate and said outer laminate;
an intravascular catheter slidably disposed within said tubular body, said catheter having an intravascular stent mounted thereon;
a manipulating device attached to a proximal end of said tubular body for effecting relative axial movement of said catheter with respect to said tubular body; and
a passageway formed in a distal end of said tubular body to allow a portion of said catheter carrying said stent to pass therethrough.

2. The stent delivery system of claim 1, wherein said spiral-wound ribbon has a predetermined number of turns to provide said tubular body with lateral flexibility.

3. The stent delivery system of claim 2, wherein said predetermined-number-of-turns ranges from 19.7 turns per centimeter to 27.6 turns per centimeter (50 turns per inch to 70 turns per inch).

4. The stent delivery system of claim 1, wherein said spiral-wound ribbon is made from an inelastic material to provide said tubular body with longitudinal stiffness and lateral flexibility.

5. The stent delivery system of claim 1, wherein said inner laminate and said outer laminate are made from polyimide to provide said tubular body with longitudinal stiffness and lateral flexibility.

6. The stent delivery system of claim 1, wherein said tubular body further comprises a thin-walled material that encapsulates said inner laminate and said outer laminate.

7. The stent delivery system of claim 1, wherein the distal portion is defined by said inner laminate, said outer laminate, and said spiral-wound ribbon encapsulated between said inner laminate and said outer laminate.

8. The stent delivery system of claim 7, further comprising a port formed in said tubular body, said port adapted for receiving a guidewire therethrough.

9. The stent delivery system of claim 8, further comprising a slot formed in said tubular body, said slot extending from said port to a point proximate said passageway, said slot adapted for facilitating the relative movement of the guidewire and the tubular body.

10. The stent delivery system of claims 7 or 8, further comprising a plurality of slits formed in said distal end, said slits being substantially parallel to a long axis of the tubular body.

11. The stent delivery system of claims 7 or 8, wherein said distal end tapers to provide a low profile for the tubular body.

12. The stent delivery system of claims 7 or 8, wherein said distal end has at least one notch to increase flexibility.

13. The stent delivery system of claims 7 or 8, further comprising a thin-walled material that encapsulates said inner laminate and said outer laminate.
